# EUROPEAN PATENT APPLICATION

(11) **EP 3 545 756 A1**
(43) Date of publication of application: **02.10.2019**
(21) Application number: 18164815.5
(22) Date of filing: 28.03.2018
(51) Int. Cl.: A01H 4/00, A01H 1/08, C12N 5/00, C12N 15/82

(54) **REGENERATION OF PLANTS IN THE PRESENCE OF INHIBITORS OF THE HISTONE METHYLTRANSFERASE EZH2**

(71) Applicant: KWS SAAT SE & Co. KGaA, 37574 Einbeck (DE)
(72) Inventor: Kong, Jixiang, Einbeck 37574 (DE)

(57) **Abstract**

The present invention relates to the field of plant breeding and engineering and in particular to the regeneration of plants from cells and other tissues. More particularly, the invention provides methods and means for improving shoot formation and regeneration of plants using a histone methyltransferase EZH2 inhibitor.

## Description

The present invention relates to the field of plant breeding and in particular to the regeneration of plants from cells and other tissues. More particularly, the invention provides methods and means for improving shoot formation and regeneration of plants using an inhibitor of the histone methyltransferase EZH2.

Plant regeneration involves the *in vitro* culture of cells, tissues, and organs under defined physical and chemical conditions. Regeneration has long been known to occur in plants. In plants differentiated cells are able to regenerate into the full array of tissues under appropriate culture conditions. Regeneration can involve direct or indirect organogenesis. In direct regeneration, *in vitro* organs are directly induced from explant tissues; in indirect regeneration, a *de novo* organ is typically formed from an intermediate tissue, the callus. Plant calli are undifferentiated structures that can give rise to new tissues. Plant leaves, shoots, roots, and embryos can variously be elicited from a growing callus by treating it with different ratios of hormones.

Generally, three phases can be recognized throughout plant regeneration. First, somatic cells of explant tissues can respond to hormonal signals to acquire features similar to meristematic cells, a process known as "dedifferentiation". Second, callus cells with organogenic competence are reprogrammed and determined for specific organ formation under the influence of hormone balance. The third regeneration phase, morphogenesis, is independent of exogenously supplied hormones. Thus, exogenous hormone treatment is the critical factor triggering early developmental events in *in vitro* regeneration.

However, obtaining dedifferentiated cells (callus) that can regenerate into whole plants is not always feasible for many plant species. Sugar beet is known to be recalcitrant for dedifferentiation and plant regeneration. These difficulties were major obstacles for obtaining transgenic sugar beets for example through an *Agrobacterium*-mediated transformation procedure. Since decades breeders and researchers are working on the development of more efficient protocols for transformation and regeneration of plants recalcitrant to callus formation. Typically, such plants show genotypic variations causing drastic differences of rates of callus and shoot formation between different lines (Ivic-Haymes & Smigocki (2005), "Identification of highly regenerative plants within sugar beet (Beta vulgaris L.) breeding lines for molecular breeding." In Vitro Cellular and Developmental Biology-Plant, 41(4), 483-488; Mishutkina & Gaponenko (2006), "Sugar beet (Beta vulgaris L.) morphogenesis in vitro: effects of phytohormone type and concentration in the culture medium, type of explants, and plant genotype on shoot regeneration frequency." Russian Journal of Genetics, 42(2), 150-157; Tomita et al. (2013), "Evaluation of the potential for somatic embryogenesis in sugar beet (Beta vulgaris L.) breeding lines and improvement of regeneration efficiency." Plant Biotechnology, 30(5), 479-487.). Often regeneration for certain genotypes is not feasible at all. Kishchenko et al. 2005 ("Production of transgenetic sugarbeet (Beta vulgaris L.) plants resistant to phosphinothricin." Cell biology international, 29(1), 15-19.) and Kagami at el. 2015 ("Sugar beet (Beta vulgaris L.)." Agrobacterium Protocols: Volume 1, 335-347.) disclose well-known protocols for the transformation of sugar beet, however these protocols show strong genotype dependency.

DZNep (3-Deazaneplanocin A hydrochloride) has been mainly used in cancer research to modify histone modification in order to activate silenced genes in cancer cells (Miranda, T. B. et al. (2009). DZNep is a global histone methylation inhibitor that reactivates developmental genes not silenced by DNA methylation. Molecular cancer therapeutics, 8(6), 1579-1588.). Treatment of DZNep in cancer cells could lead to apoptosis, which makes it a potential candidate for cancer treatment (Girard, N. et al. (2014). 3-Deazaneplanocin A (DZNep), an inhibitor of the histone methyltransferase EZH2, induces apoptosis and reduces cell migration in chondrosarcoma cells. PloS one, 9(5), e98176.). In addition, DZNep was discovered in a chemical screen that can activate Oct4 expression, which provides a strategy to induce pluripotent stem cells from somatic cells in clinical applications (Hou, P. et al. (2013). Pluripotent stem cells induced from mouse somatic cells by small-molecule compounds. Science, 341(6146), 651-654.). The general mechanism of DZNep effect was demonstrated to be the change of histone modification such as H3K27me3 by inhibiting histone methyltransferase. There is not much known about the effect of DZNep in plants except one report using DZNep to activate silenced promoter in Arabidopsis (Foerster, A. M. et al. (2011). Genetic rearrangements can modify chromatin features at epialleles. PLoS genetics, 7(10), e1002331).

Surprisingly, the inventors found that inhibitors of the histone methyltransferase EZH2 like 3-Deazaneplanocin A (DZNep) hydrochloride have a positive effect on shoot regeneration from calli for example of Beta vulgaris. Such effect of DZNep and other inhibitors of the histone methyltransferase EZH2 has not been proved for indirect regeneration protocols or to overcome recalcitrance in certain plant species like Beat vulgaris or other plants before. Importantly, neither DZNep nor any other inhibitor of the histone methyltransferase EZH2 has been used in any transformation protocol of crop, aiming to improve the efficiency.

Thus, a first aspect of the present invention is the use of an inhibitor of the histone methyltransferase EZH2, in particular DZNep and salts or derivatives thereof, in a method for inducing shoot formation from callus tissue of a plant.

The present invention provides a method for inducing shoot formation from callus tissue of a plant, comprising the step of cultivating the callus tissue in the presence of an inhibitor of the histone methyltransferase EZH2 in particular DZNep and salts or derivatives thereof.

Cultivating callus tissue may comprise growing the callus tissue in a medium comprising an inhibitor of the histone methyltransferase EZH2. Alternatively or additionally, the inhibitor of the histone methyltransferase EZH2 can be introduced into cells of the callus tissue, for example via bombardment, electroporation or microinjection or any other method known to the skilled person. According to the invention it is preferred to grow the callus tissue in a medium containing an inhibitor of the histone methyltransferase EZH2, in particular DZNep and salts or derivatives thereof. The cultivating step can be carried out using any medium well-known in the art for growing callus tissue. Depending on the plant in question, these conditions may vary. In principle, several types of basal salt mixtures can be used for culturing callus tissue, but most preferred, the medium comprises modified Murashige and Skoog medium, White's medium, or woody plant medium.

According to a preferred aspect of the invention, the medium is supplemented with the inhibitor of the histone methyltransferase EZH2. The concentration of the inhibitor of the histone methyltransferase EZH2, in particular DZNep and salts or derivatives thereof, in the medium can range from about 0.05 µM up to about 5.0 µM. In order to achieve the desired boost of shoot formation, the concentration of the inhibitor of the histone methyltransferase EZH2 in the medium is preferably in a range of 0.1 µM to 2.0 µM.

In addition to the inhibitor of the histone methyltransferase EZH2, one or more further additives can be used in the culture medium. For example, the culture medium can be supplemented with plant growth regulators, such as auxins, cytokinins, and gibberellins, to initiate callus formation. Vitamins can be provided to enhance growth, such as Gamborg B5 vitamins. Enrichment with nitrogen, phosphorus and potassium also proved to be helpful.

In terms of the invention, "inhibitor of the histone methyltransferase EZH2" or "EZH2-inhibitor" refers to any chemical compound that inhibits the histone methyltransferase EZH2. It is understood, that the inhibitor of the histone methyltransferase EZH2 can be a single compound or a combination of several compounds. A preferred class of compounds suitable to provide the desired histone methyltransferase inhibitory activity is 3-deazaneplanocin A (DZNep), in particular in form of the hydrochloride as well as any other salts and analogues or derivatives thereof. Suitable derivatives are described for example in WO2010/036213. Derivatives include for example aristeromycin, 3-deazaaristeromycin hydrochloride, (1S,2R,5R)-5-(6-amino-9H-purin-9-yl)-3-(methoxymethyl)cyclopent-3-ene-1,2-diol hydrochloride, (1S,2R,5R)-5-(6-amino-9H-purin-9-yl)-3-(fluoromethyl)cyclopent-3-ene-1,2-diol) hydrochloride or (1R,2S,3R)-3-(6-amino-9H-purin-9-yl)cyclopentane-1,2-diol, (1R,2S,3R)-3-(4-amino-1H-imidazo[4,5-c]pyridin-1-yl)cyclopentane-1,2-diol, (1R, 2S, 3R)-3-(6-amino-9H-purin-9-yl)-1,2-cyclopentanediol hydrochloride, 2',3'-O-isopropylidene-3-deazaneplanocin A or (1S,2R,5R)-5-(6-amino-9H-purin-9-yl)-3-methylcyclopent-3-ene-1,2-diol hydrochloride or an enantiomer or diastereomer thereof or a salt of any of these.

Surprisingly, it was found that inhibitors of histone methyltransferase EZH2, in particular DZNep and salts or derivatives thereof, are suitable for inducing shoot formation even in recalcitrant plant species or plant genotypes. Thus, using the approach of the invention it is possible to improve indirect regeneration in recalcitrant plant species or plant genotypes.

Callus tissue for use in the method of the present invention can be provided using any method known in the art. For example, callus tissue can be provided by inducing callus formation from at least one plant cell. Accordingly, the present invention provides a method for regenerating shoots from a plant cell, comprising the following steps:
(a) Inducing callus formation from at least one plant cell under conditions promoting the growing of callus tissue, and
(b) Cultivating the callus tissue obtained in step (a) as described herein above.

In principle, it is sufficient to use only one plant cell for inducing callus formation. Thus, if the plural "plant cells" is used in the following the wording must not be understood in that a minimum number of plant cells would be required.

Plant cells suitable for inducing callus tissue include embryonic plant cells and somatic plant cells. The way how these plant cells are provided is not important for the method according to the present invention. For example, embryonic or somatic plant cells can be provided from an explant isolated from a plant. Which part of a plant is eligible for obtaining an explant depends on the particular plant species. Generally, suitable plant cells can be obtained from hypocotyl, shoot, leaves, buds, flowers and roots of a plant.

According to a preferred aspect of the invention, callus tissue is provided by a method of inducing callus formation from at least one plant cell comprising the step of cultivating the at least one plant cell in the presence of a histone deacetylase inhibitor (HDACi) like trichostatin A (TSA).

According to another aspect of the invention, the beneficial effect of an inhibitor of the histone methyltransferase EZH2, in particular DZNep and salts or derivatives thereof, on shoot formation can be exploited in methods of producing transgenic plants as well as in methods for producing genetically modified plants. It was found, that in recalcitrant plant species or plant genotypes, transformation efficiency can be improved by using an inhibitor of the histone methyltransferase EZH2. The regeneration of plants from modified plant cells that have been transformed or gene edited and possibly have a modified genome is significantly improved when using an inhibitor of the histone methyltransferase EZH2 in the step of shoot formation.

Accordingly, the invention provides a method for producing a transgenic plant, comprising the following steps:
(a) Inducing callus formation from at least one plant cell under conditions promoting the growing of callus tissue,
(b) Introducing into a plant cell to be used in step (a) and/or into a cell of the callus obtained in step (a) at least one nucleotide sequence of interest or at least one polypeptide of interest,
(c) Cultivating the callus tissue obtained from steps (a) and (b) as described above, and
(d) Regenerating a transgenic plant.

Step (a) of inducing callus formation can be performed using any method known in the art. By way of example, callus formation can be induced in the presence of a TSA which can be added to the medium or directly into the plant cells.

In step (b), a cell is transformed by introducing a nucleic acid molecule into the cell in a manner to cause stable or transient presence of the nucleic acid sequence, preferably stable or transient expression of the nucleic acid sequence or by introducing a polypeptide into the cell in a manner to cause transient presence. Stable presence of for example a DNA sequence means that said DNA sequence is stable incorporated into the genome of the cell. Stable expression refers to the expression of for example a DNA sequence that is stable incorporated into the genome of the cell. Transformation of both monocotyledonous and dicotyledonous plant cells is now routine, and the selection of the most appropriate transformation technique will be determined by the practitioner. The choice of method will vary with the type of plant to be transformed; those skilled in the art will recognize the suitability of particular methods for given plant types. Suitable methods can include, but are not limited to: electroporation of plant protoplasts; liposome-mediated transformation; polyethylene glycol (PEG) mediated transformation; transformation using viruses; micro-injection of plant cells; micro-projectile bombardment of plant cells; vacuum infiltration; and Agrobacterium-mediated transformation.

According to one embodiment of the present invention, the at least one nucleotide sequence of interest or at least one polypeptide of interest is introduced into the plant cell to be used in step (a) of inducing callus formation. It is understood that in this case step (b) is carried out before step (a). According to another embodiment of the invention the at least one nucleotide sequence of interest or at least one polypeptide of interest is introduced into a cell of the callus obtained in step (a). It is understood that in this case, step (b) is carried out after step (a). Additionally, it is possible to introduce nucleotide sequences of interest or polypeptides of interest both into the cell to be used for callus formation and into the cell of the callus resulting from step (a). According to this embodiment the method includes the following steps:
(i) introducing into a plant cell at least one nucleotide sequence of interest or at least one polypeptide of interest,
(ii) inducing callus formation from the cell obtained in step (i) and
(iii) introducing at least one nucleotide sequence of interest or at least one polypeptide of interest into a cell of the callus obtained in step (ii).

The step of introducing the at least one nucleotide sequence of interest or at least one polypeptide of interest can be performed using any suitable method commonly known in the art. A number of methods as also mentioned above is available to transfer nucleic acids of interest or polypeptides into plant cells. An exemplary vector mediated method for introducing DNA molecules is Agrobacterium-mediated transformation, as described, for example, by Lindsay & Gallois, 1990, Journal of Experimental Botany, and Kischenko et al., 2005, Cell Biology International for sugar beet, by Ishida et al., 2007, ("Agrobacterium-mediated transformation of maize." Nature protocols, 2(7), 1614-1621) for corn, or by the PureWheat Technology from Japan Tobacco company for wheat. Other suitable techniques also suitable for introducing RNA molecules or polypeptides include particle bombardment and electroporation.

The nucleotide sequence of interest according to the invention may be a DNA or RNA sequence, e.g. mRNA, siRNA, miRNA, tRNA etc. More particularly, the nucleotide sequence of interest may encode a polypeptide conferring at least one phenotypic trait. Preferably, the phenotypic trait conferred by the polypeptide can be selected from the group consisting of resistance/tolerance to biotic stress, including pathogen resistance/tolerance, wherein the pathogen can be a virus, bacterial, fungal or animal pathogen, resistance/tolerance to abiotic stress including chilling resistance/tolerance, drought stress resistance/tolerance, osmotic resistance/tolerance, heat stress resistance/tolerance, cold or frost stress resistance/tolerance, oxidative stress resistance/tolerance, heavy metal stress resistance/tolerance, salt stress or water logging resistance/tolerance, lodging resistance/tolerance, shattering resistance/tolerance, or resistance/tolerance against one or more herbicides like glyphosate, glufosinate, 2,4-D, Dicamba, ALS inhibitors et cetera. The at least one phenotypic trait of interest can also be selected from the group consisting of the modification of a further agronomic trait of interest including yield increase, flowering time modification, seed color modification, endosperm composition modification, nutritional content modification or metabolic engineering of a pathway of interest. Furthermore, the nucleotide sequence of interest may encode or may be a component of a gene editing machinery, e.g. a gRNA, a crRNA, a tracrRNA, a sgRNA, a nuclease, a nickase, a TALE recognition domain or a zinc finger recognition domain or a fusion protein comprising such recognition domain, etc.

A nucleic acid (molecule) or nucleotide (sequence) or polynucleotide, as used herein, refers to both DNA and RNA. DNA also includes cDNA and genomic DNA.

A nucleic acid molecule can be single- or double-stranded, and can be synthesized chemically or produced by biological expression in vitro or even in vivo.

It will be clear that whenever nucleotide sequences of RNA molecules are defined by reference to nucleotide sequence of corresponding DNA molecules, the thymine (T) in the nucleotide sequence should be replaced by uracil (U). Whether reference is made to RNA or DNA molecules will be clear from the context of the application.

The polypeptide of interest according to the invention may be a transcription factor, a protein component of a gene editing machinery, like a nuclease, a nickase, a base editor, a recognition domain of a TAL effector or a zinc finger effector, or may be a polypeptide conferring phenotypic trait which can be selected from the group consisting of resistance/tolerance against one or more herbicides like glyphosate, glufosinate, 2,4-D, Dicamba, ALS inhibitors, color marker, fluorescence marker, resistance/tolerance to biotic stress, including pathogen resistance/tolerance, wherein the pathogen can be a virus, bacterial, fungal or animal pathogen, resistance/tolerance to abiotic stress including chilling resistance/tolerance, drought stress resistance/tolerance, osmotic resistance/tolerance, heat stress resistance/tolerance, cold or frost stress resistance/tolerance, oxidative stress resistance/tolerance, heavy metal stress resistance/tolerance, salt stress or water logging resistance/tolerance, lodging resistance/tolerance, shattering resistance/tolerance, or et cetera. A polypeptide of interest can be synthesized chemically or produced by biological expression in vitro or even in vivo.

In steps (c) and (d) of the above method for producing a transgenic plant, the modified callus tissue including plant cells that have been transformed are regenerated into a whole (fertile) plant. Therefore, in step (c) the callus tissue is cultivated in the presence of an inhibitor of the histone methyltransferase EZH2 as described in detail above to induce shoot formation and finally regenerated into a whole plant in step (d).

Regeneration techniques rely on manipulation of certain phytohormones in a tissue culture growth medium, occasionally relying on a biocide and/or herbicide marker that can been introduced together with the desired nucleotide sequence(s) of interest. Plant regeneration from cultured protoplasts is described in Evans et al., Protoplasts Isolation and Culture, Handbook of Plant Cell Culture, pp. 124-176, MacMillilan Publishing Company, New York, 1983; and Binding, Regeneration of Plants, Plant Protoplasts, pp. 21-73, CRC Press, Boca Raton, 1985. Regeneration can also be obtained from plant callus, explants, protoplasts, immature or mature embryos, embryonic tissue, meristematic tissues, organs, or parts thereof. Such regeneration techniques are described generally in Klee (1987) Ann. Rev. of Plant Phys. 38:467486. To obtain whole plants from transgenic tissues such as immature embryos, they can be grown under controlled environmental conditions in a series of media containing nutrients and hormones, a process known as tissue culture. Once whole plants are generated and produce seed, evaluation of the progeny begins.

Further, the invention also provides a method for producing a genetically modified plant, comprising the following steps
(a) Inducing callus formation from at least one plant cell as described above,
(b) Modifying the genome of a plant cell to be used in step (a) and/or of a cell of the callus tissue obtained in step (a) by introducing into said cell a double stranded DNA break (DSB) inducing enzyme which preferably recognizes a predetermined site in the genome of said cell and optionally a repair nucleic acid molecule, and/or a base editor fused to a catalytically impaired double stranded DNA break (DSB) inducing enzyme which preferably recognizes a predetermined site in the genome of said cell,
   wherein the modification of said genome is selected from
   i. a replacement of at least one nucleotide;
   ii. a deletion of at least one nucleotide;
   iii. an insertion of at least one nucleotide; or
   iv. any combination of i. - iii,
(c) Cultivating the callus tissue obtained from steps (a) and (b), and
(d) Regenerating a genetically modified plant.

Step (a) of inducing callus formation is performed by the method described herein above. Preferably, callus formation is induced in the presence of TSA as the HDACi; which can be added to the medium or directly into the plant cells.

In step (b), modifying the genome of the cell is accomplished by means of a double-stranded DNA break (DSB) inducing enzyme which preferably recognizes a predetermined site in the genome of said cell.

The step of modifying the genome can be carried out before and/or after induction of callus formation. Thus, according to a first aspect of the invention, the genome of a plant cell is modified as described in step (b) and the resulting modified plant cell is then used in a subsequent step (a) of inducing callus formation. According to another aspect of the invention, step (a) of inducing callus formation is carried out first and subsequently at least one cell of the resulting callus tissue is modified in step (b) by means of a double-stranded DNA break-inducing enzyme. Furthermore, it is possible to modify the genome of both the plant cell to be used in the step of callus formation and a cell of the callus tissue resulting from the step of inducing callus formation. According to this aspect of the invention, the method includes the steps of
(i) modifying the genome of a plant cell,
(ii) inducing callus formation from the cell resulting from step (i) and
(iii) modifying the genome of a cell of the callus tissue obtained in step (ii).

As used herein, a *"double-stranded DNA break inducing enzyme"* or *"DSBI enzyme"* is an enzyme capable of inducing a double-stranded DNA break at a particular nucleotide sequence, called the *"recognition site".* The double-stranded DNA break (DSB)-inducing enzyme can, for example, be selected from the group consisting of meganuclease, TAL effector nuclease, zinc finger nuclease, CRISPR systems like CRISPR/Cas9, CRISPR/Cpf1, CRISPR/CasX or CRISPR/CasY. Rare-cleaving endonucleases are DSBI enzymes that have a recognition site of preferably about 14 to 70 consecutive nucleotides, and therefore have a very low frequency of cleaving, even in larger genomes such as most plant genomes. Homing endonucleases, also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double strand break in the intronless or inteinless alleles, these nucleases create recombinogenic ends, which engage in a gene conversion process that duplicates the coding sequence and leads to the insertion of an intron or an intervening sequence at the DNA level. A list of other rare cleaving meganucleases and their respective recognition sites is provided in Table I of WO 03/004659 (pages 17 to 20) (incorporated herein by reference).

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the non-specific DNA-cleavage domain from a natural restriction enzyme, such as Fokl. Such methods have been described e.g. in WO 03/080809, WO 94/18313 or WO 95/09233 and in Isalan et al., 2001, Nature Biotechnology 19, 656- 660; Liu et al. 1997, Proc. Natl. Acad. Sci. USA 94, 5525-5530).

Another example of custom-designed endonucleases includes the so-called TALE nucleases (TALENs), which are based on transcription activator-like effectors (TALEs) from the bacterial genus Xanthomonas fused to the catalytic domain of a nuclease (e.g. Fokl or a variant thereof). The DNA binding specificity of these TALEs is defined by repeat-variable diresidues (RVDs) of tandem-arranged 34/35-amino acid repeat units, such that one RVD specifically recognizes one nucleotide in the target DNA. The repeat units can be assembled to recognize basically any target sequences and fused to a catalytic domain of a nuclease create sequence specific endonucleases (see e.g. Boch et al., 2009, Science 326:p1509-1512; Moscou and Bogdanove, 2009, Science 326:p1501; and WO 2010/079430, WO 2011/072246, WO 2011/154393, WO 2011/146121, WO 2012/001527, WO 2012/093833, WO 2012/104729, WO 2012/138927, WO 2012/138939). WO2012/138927 further describes monomeric (compact) TALENs and TALENs with various catalytic domains and combinations thereof.

Recently, a new type of customizable endonuclease system has been described; the so-called CRISPR/Cas system. A CRISPR system in its natural environment describes a molecular complex comprising at least one small and individual non-coding RNA in combination with a Cas nuclease or another CRISPR nuclease like a Cpf1 nuclease (Zetsche et al., "Cpf1 Is a Single RNA-Guides Endonuclease of a Class 2 CRISPR-Cas System", Cell, 163, pp. 1-13, October 2015) which can produce a specific DNA double-stranded break. Presently, CRISPR systems are categorized into 2 classes comprising five types of CRISPR systems, the type II system, for instance, using Cas9 as effector and the type V system using Cpf1 as effector molecule (Makarova et al., Nature Rev. Microbiol., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova *et al.,* 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as key enzyme for the interference step, which system contains both a crRNA and also a transactivating RNA (tracrRNA) as the guide motif. These hybridize and form double-stranded (ds) RNA regions which are recognized by RNAseIII and can be cleaved in order to form mature crRNAs. These then in turn associate with the Cas molecule in order to direct the nuclease specifically to the target nucleic acid region. Recombinant gRNA molecules can comprise both the variable DNA recognition region and also the Cas interaction region and thus can be specifically designed, independently of the specific target nucleic acid and the desired Cas nuclease. As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "NGG" or "NAG" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "NNGRRT" or "NNGRR(N)". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpf1 nucleases it has been described that the Cpf1-crRNA complex, without a tracrRNA, efficiently recognize and cleave target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized. Further CRISPR effectors like CasX and CasY effectors originally described for bacteria, are meanwhile available and represent further effectors, which can be used for genome engineering purposes (Burstein et al., "New CRISPR-Cas systems from uncultivated microbes", Nature, 2017, 542, 237-241).

The cleavage site of a DSBI enzyme relates to the exact location on the DNA where the double-stranded DNA break is induced. The cleavage site may or may not be comprised in (overlap with) the recognition site of the DSBI enzyme and hence it is said that the cleavage site of a DSBI enzyme is located at or near its recognition site. The recognition site of a DSBI enzyme, also sometimes referred to as binding site, is the nucleotide sequence that is (specifically) recognized by the DSBI enzyme and determines its binding specificity. For example, a TALEN or ZNF monomer has a recognition site that is determined by their RVD repeats or ZF repeats respectively, whereas its cleavage site is determined by its nuclease domain (e.g. Fokl) and is usually located outside the recognition site. In case of dimeric TALENs or ZFNs, the cleavage site is located between the two recognition/binding sites of the respective monomers, this intervening DNA region where cleavage occurs being referred to as the spacer region.

A person skilled in the art would be able to either choose a DSBI enzyme recognizing a certain recognition site and inducing a DSB at a cleavage site at or in the vicinity of the preselected site or engineer such a DSBI enzyme. Alternatively, a DSBI enzyme recognition site may be introduced into the target genome using any conventional transformation method or by crossing with an organism having a DSBI enzyme recognition site in its genome, and any desired DNA may afterwards be introduced at or in the vicinity of the cleavage site of that DSBI enzyme.

In a particularly preferred aspect of this embodiment, a repair nucleic acid molecule is additionally introduced into the plant cell.

As used herein, a *"repair nucleic acid molecule"* is a single-stranded or double-stranded DNA molecule or RNA molecule that is used as a template for modification of the genomic DNA at the preselected site in the vicinity of or at the cleavage site. As used herein, *"use as a template for modification of the genomic DNA",* means that the repair nucleic acid molecule is copied or integrated at the preselected site by homologous recombination between the flanking region(s) and the corresponding homology region(s) in the target genome flanking the preselected site, optionally in combination with non-homologous end-joining (NHEJ) at one of the two end of the repair nucleic acid molecule (e.g. in case there is only one flanking region). Integration by homologous recombination will allow precise joining of the repair nucleic acid molecule to the target genome up to the nucleotide level, while NHEJ may result in small insertions/deletions at the junction between the repair nucleic acid molecule and genomic DNA.

A "base editor" as used herein refers to a protein or a fragment thereof having the capacity to mediate a targeted base modification, i.e., the conversion of a base of interest resulting in a point mutation of interest. Preferably, the at least one base editor in the context of the present invention is temporarily or permanently fused to at least one DSBI enzyme, or optionally to a component of at least one DSBI. The fusion can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al ((2017). Programmable base editing of A•T to G•C in genomic DNA without DNA cleavage. Nature, 551(7681), 464.) described adenine base editors (ABEs) that mediate the conversion of A•T to G•C in genomic DNA.

As used herein, "a *modification of the genome",* means that the genome has changed by at least one nucleotide. This can occur by replacement of at least one nucleotide and/or a deletion of at least one nucleotide and/or an insertion of at least one nucleotide, as long as it results in a total change of at least one nucleotide compared to the nucleotide sequence of the preselected genomic target site before modification, thereby allowing the identification of the modification, e.g. by techniques such as sequencing or PCR analysis and the like, of which the skilled person will be well aware.

As used herein "a *preselected site"* or *"predefined site"* indicates a particular nucleotide sequence in the genome (e.g. the nuclear genome) at which location it is desired to insert, replace and/or delete one or more nucleotides. This can e.g. be an endogenous locus or a particular nucleotide sequence in or linked to a previously introduced foreign DNA or transgene. The preselected site can be a particular nucleotide position at (after) which it is intended to make an insertion of one or more nucleotides. The preselected site can also comprise a sequence of one or more nucleotides which are to be exchanged (replaced) or deleted.

As used in the context of the present application, the term *"about"* means +/- 10% of the recited value, preferably +/- 5% of the recited value. For example, about 100 nucleotides (nt) shall be understood as a value between 90 and 110 nt, preferably between 95 and 105.

As used herein, a *"flanking region",* is a region of the repair nucleic acid molecule having a nucleotide sequence which is homologous to the nucleotide sequence of the DNA region flanking (i.e. upstream or downstream) of the preselected site. It will be clear that the length and percentage sequence identity of the flanking regions should be chosen such as to enable homologous recombination between said flanking regions and their corresponding DNA region upstream or downstream of the preselected site. The DNA region or regions flanking the preselected site having homology to the flanking DNA region or regions of the repair nucleic acid molecule are also referred to as the homology region or regions in the genomic DNA.

To have sufficient homology for recombination, the flanking DNA regions of the repair nucleic acid molecule may vary in length, and should be at least about 10 nt, about 15 nt or about 20 nt in length. However, the flanking region may be as long as is practically possible (e.g. up to about 100-150 kb such as complete bacterial artificial chromosomes (BACs). Preferably, the flanking region will be about 50 nt to about 2000 nt, e.g. about 100 nt, 200 nt, 500 nt or 1000 nt. Moreover, the regions flanking the DNA of interest need not be identical to the homology regions (the DNA regions flanking the preselected site) and may have between about 80% to about 100% sequence identity, preferably about 95% to about 100% sequence identity with the DNA regions flanking the preselected site. The longer the flanking region, the less stringent the requirement for homology. Furthermore, to achieve exchange of the target DNA sequence at the preselected site without changing the DNA sequence of the adjacent DNA sequences, the flanking DNA sequences should preferably be identical to the upstream and downstream DNA regions flanking the preselected site.

As used herein, *"upstream"* indicates a location on a nucleic acid molecule which is nearer to the 5' end of said nucleic acid molecule. Likewise, the term *"downstream"* refers to a location on a nucleic acid molecule which is nearer to the 3' end of said nucleic acid molecule. For avoidance of doubt, nucleic acid molecules and their sequences are typically represented in their 5' to 3' direction (left to right).

In order to target sequence modification at the preselected site, the flanking regions must be chosen so that 3' end of the upstream flanking region and/or the 5' end of the downstream flanking region align(s) with the ends of the predefined site. As such, the 3' end of the upstream flanking region determines the 5' end of the predefined site, while the 5' end of the downstream flanking region determines the 3' end of the predefined site.

As used herein, said preselected site being located outside or away from said cleavage (and/or recognition) site, means that the site at which it is intended to make the genomic modification (the preselected site) does not comprise the cleavage site and/or recognition site of the DSBI enzyme, i.e. the preselected site does not overlap with the cleavage (and/or recognition) site. Outside/away from in this respect thus means upstream or downstream of the cleavage (and/or recognition) site.

Steps (c) and (d) of the above method for producing a genetically modified plant are carried out as described above in the context of producing a transgenic plant. Thus, the modified plant cell that has been gene edited and possibly has a modified genome is regenerated into a whole (fertile) plant.

Still a further aspect of the present invention relates to the production of haploid and double haploid plant embryos and plants. It was found that the beneficial effect of EZH2-inhibitors on shoot formation can also be exploited for producing haploid and double haploid plant embryos. Thus, the present invention provides a method for producing a haploid plant embryo, comprising the steps
(a) Inducing callus formation from an immature male gametophyte or a microspore under conditions promoting the growing of callus tissue,
(b) Cultivating the callus tissue obtained in step (a) as described herein above to form shoots, and
(c) Optionally conducting chromosome doubling.

The haploid or double haploid plant embryo obtained in this manner can then be regenerated into a mature plant. Thus, the present invention also provides a method for producing a haploid or double haploid plant, comprising the steps
(a) Inducing callus formation from an immature male gametophyte or a microspore under conditions promoting the growing of callus tissue,
(b) Cultivating the callus tissue obtained in step (a) as described herein above to form shoots,
(c) Optionally conducting chromosome doubling, and
(d) Regenerating a haploid or double haploid plant.

Haploids are plants (sporophytes) that contain a gametic chromosome number. Spontaneous haploid individuals have been identified in several plant species. However, spontaneous evidence is a rare event, resulting in a limited application. Hence artificial haploid induction is necessary for potential use in breeding. Induction protocols substantially vary, not only among species but also among genotypes of the same species.

In the methods of the present invention, immature male gametophytes or microspores are used as the starting material for producing haploids and double haploids. In step (a), callus formation is induced under conditions promoting the growing of callus tissue. The method relies on the ability of immature male gametophytes and microspores to convert their developmental pathway from gametophytic (leading to mature pollen grain) to sporophytic, resulting in cell division at a haploid level followed by formation of calluses and embryos. This process can be induced by several factors. The most widely used triggering factors are temperature pre-treatment, sucrose and nitrogen starvation and osmotic stress. In addition to stress treatments, the culture media constituents have a significant effect. Depending on the plant concerned, a skilled person can choose among a variety of well-known methods suitable to induce callus formation from immature male gametophytes or microspores.

In step (b), the callus tissue is cultivated in the presence of an inhibitor of the histone methyltransferase EZH2, in particular DZNep or a salt or derivative thereof, as described in detail herein above, to induce shoot formation.

Due to the absence of one set of homologous chromosomes in haploid plants, meiosis cannot occur, so there is no seed set. Therefore, duplication of the chromosome complement can be accomplished in step (c) of the above methods for producing haploid and double haploid plant embryos and plants. Various techniques have been applied over several decades and are well known to the skilled person. The most frequently used application is treatment with anti-microtubule drugs, such as colchicine (originally extracted from autumn crocus *Colchicum autumnale),* which inhibits microtubule polymerization by binding to tubulin. Although colchicine is highly toxic, used at a millimolar concentration and known to be more efficient in animal than in plant tissues, it is still the most widely used doubling agent. Other options are oryzalin, amiprophosmethyl (APM), trifluralin and pronamide, all of which are used as herbicides and are effective in micromolar concentrations.

Anti-microtubule drugs might be applied at various stages of the above methods, such as being incorporated into microspore pretreatment media. The treatment of plants at later developmental stages has the advantage that only already tested haploid regenerants are treated either *in vitro* (for instance at the shoot culture stage) or *in vivo* following acclimatization. The concentration and duration of treatments must be always determined in relation to two effects: the percentage of doubled plants and the percentage of survival.

The present invention is applicable to any plant species, whether monocot or dicot. Preferably, plants which may be subject to the methods and uses of the present invention are selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Helianthus annuus, Helianthus tuberosus* and/or *Allium tuberosum.* Particularly preferred are *Beta vulgaris, Zea mays, Triticum aestivum, Hordeum vulgare, Secale cereale, Helianthus annuus, Solanum tuberosum, Sorghum bicolor, Brassica rapa, Brassica napus, Brassica juncacea,* Brassica oleracea, *Glycine max,* and/or *Gossypium sp.*

Subject-matter of the present invention are also the plants that are obtained or obtainable by the methods described above. Accordingly, one embodiment of the invention is a transgenic plant obtained or obtainable by the above method of transforming a plant cell and regenerating a plant from said cell, as well as progeny or parts thereof, wherein the progeny or the part comprises the at least one nucleotide sequence of interest as transgene. Another embodiment of the invention is a genetically modified plant obtained or obtainable by the above method of modifying the genome of a plant cell and regenerating a plant from said cell as well as progeny or parts thereof, wherein the progeny or the part comprises the modification in the genome introduced by the inventive method. Still a further embodiment of the present invention is a haploid or double haploid plant obtained or obtainable by the above method of producing a haploid plant embryo.

Further subject-matter of the present invention is a plant cell or a seed derived from the above transgenic plant or genetically modified plant. A plant cell derived from the above transgenic plant comprises the at least one nucleotide sequence of interest as transgene while a plant cell derived from the above genetically modified plant comprises the modification in its genome.

Still a further embodiment of the present invention is a plant cell or a seed derived from the above haploid or double haploid plant. The plant cell or seed comprises a haploid or double haploid set of chromosomes.

The invention will be further described with reference to the following Figures and Examples described herein. However, it is to be understood that the invention is not limited to such Examples.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Cray, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

All patents, patent applications, and publications or public disclosures (including publications on internet) referred to or cited herein are incorporated by reference in their entirety.

### Figures

**Fig.1****:** DZNep increases the efficiency of shoot formation from the sugar beet callus.
   **A:** Photographs of calli treated with 0.4 mg/l DZNep and without DZNep (control). White arrows show regenerating shoots from the callus tissue.
   **B:** Quantification of shoot regeneration includes three technical repeats for the control and DZNep treated samples.
**Fig.2****:** Vector map of plasmid pZFNnptll with the red fluorescent marker, tdT.
**Fig.3****:** DZNep increases the efficiency of shoot formation after callus bombardment.
   **A:** Photographs of calli treated with the supplement of 0.4 mg/l DZNep and with standard medium (without DZNep), bombarded and not bombarded.
   **B:** Quantification of shoot regeneration includes three technical repeats for the bombarded samples.
**Fig.4****:** Sugar beet plants with temporary DZNep treatment show fast growth.

### Examples

### 1. Shoot formation in DZNep-supplemented medium

The applied method is based on the publication by Kischenko et al. (2005. Production of transgenetic sugarbeet (Beta vulgaris L.) plants resistant to phosphinothricin. Cell biology international, 29(1), 15-19.). This method relies on the induction of friable callus derived from cotyledons and hypocotyl explants. Method is genotype dependent.

### Sugar beet callus induction protocol

1. Micropropagated shoots of the genotype 9BS0448 were used as starting material. Shoots were multiplied in MS salts supplemented with 30 g/l sucrose and 0.25 mg/l benzyladenine (BAP).
2. To induce friable callus, leaf explants were isolated from micropropagated shoots and incubated in medium containing MS salts including 15 g/l sucrose and 2 mg/l BAP at 28°C in the dark for 7 weeks.

### Technical description of the shoot regeneration protocol

3. Friable calli of step 2 were harvested and placed in medium containing MS salts, 30 g/l sucrose, 1 mg/l GA3 and 1 mg/l TDZ, and transferred to separate dishes as control. Similar amount of friable calli were harvested and placed in the same medium supplemented with 0.4 mg/l DZNep.
4. The dishes were incubated under the light/dark regime (16 h/8 h) at 24°C for 8 days.
5. Developing shoots were counted under a stereomicroscope, in order to estimate the regeneration capacity.

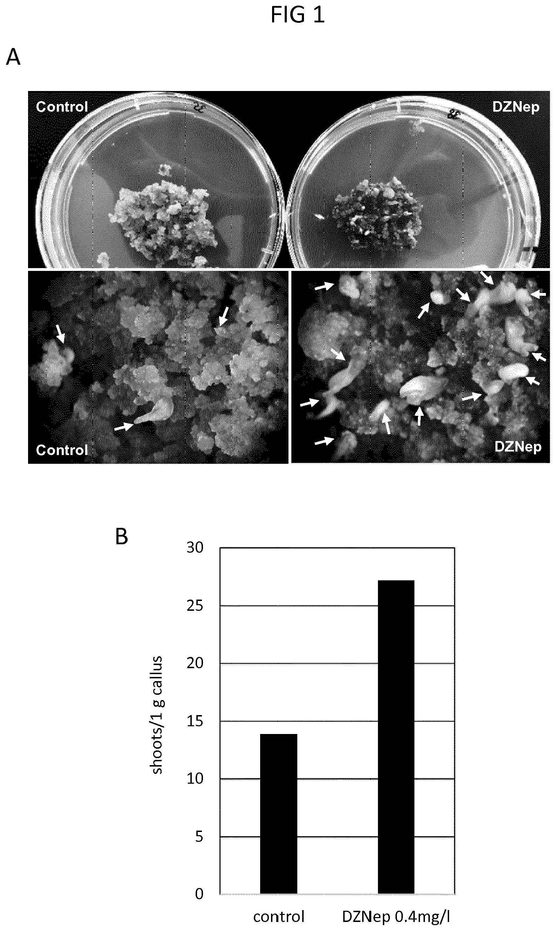

The application of the chemical DZNep in a concentration of 0.4 mg/l in the shoot induction media enhances the capability of the callus for shoot formation (Figure 1). The control shows a mean value of 13.9 shoots per 1 g callus tissue while the supplementation of DZNep results in a mean value of 27.1 shoots per 1 g callus tissue (i.e. an increase by 95%).

### 2. Particle bombardment of sugar beet callus

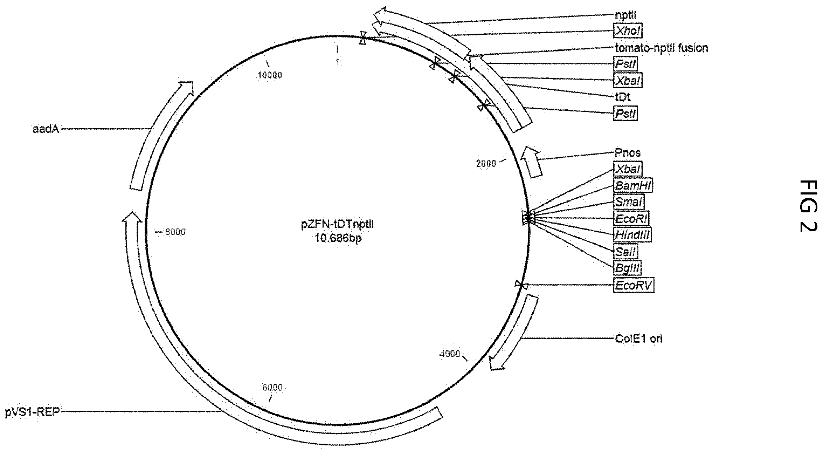

1. Friable calli were produced as described above in steps 1 and 2 of Example 1.
2. An osmotic treatment was carried out by incubating the harvested calli in a medium containing MS salts, 30 g/l sucrose,1 mg/l GA3,1 mg/l Thidiazuron (TDZ),10 g/l agar, pH 6.0, 36 g/l mannitol and 36 g/l sorbitol for 2.5 h at room temperature in the dark.
3. Preparation and DNA coating of the gold particles was done by standard procedures, as describe in the PDS-1000/He instruction manual. The plasmid pZFNnptll with the red fluorescent marker, tdT, (see Figure 2) was coprecipitated with gold particles.
4. Calli were bombarded with a PDS-1000/He unit (Bio-Rad), using 30 ng gold particles coated with 500 ng DNA per shot. The rupture pressure was 450 psi and the target tissue was placed at 9 cm distance from the stopping screen.
5. After bombardment, aliquots of calli tissue were incubated in medium containing MS salts, 30 g/l sucrose, 1 mg/l GA3 and 1 mg/l TDZ, with and without 0.4 mg/l DZNep, in light/dark (16h/8h), at 24 °C for 10 days on separate plates. As a control for standard regeneration, non-bombarded aliquots of calli were treated in the osmotic medium and directly transferred to above medium without DZNep. Shoot number was scored by using a standard binocular.

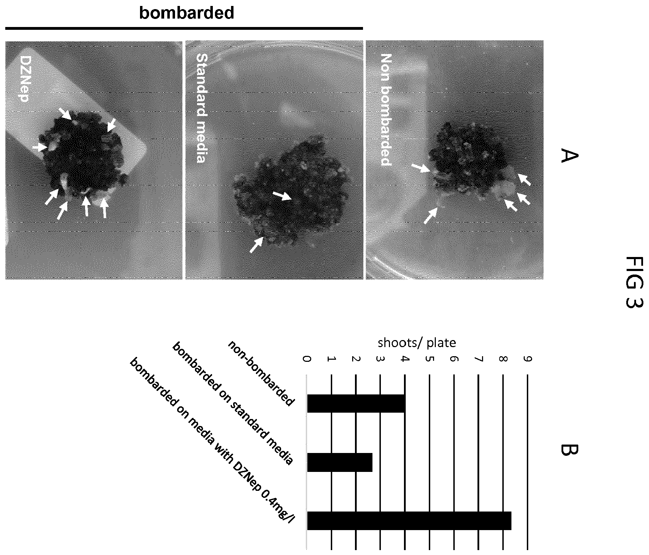

The application of the chemical DZNep in the shoot induction media increases the rate of shoot formation upon cell damage such as bombardment (Figure 3). The non-bombarded control shows a mean value of 4 shoots per plate, the bombarded control (with standard medium) a mean value of 2.7 shoots per plate. The lower value is an indicator for cell damages caused by the treatment with the particle gun. Even also bombarded the calli treated with DZNep develop the highest number of shoots, a mean value of 8.3 shoots per plate. This is an increase by 108% compared to the non-bombarded control and even by 207% compared to the bombarded control.

### 3. Plant growth after regeneration

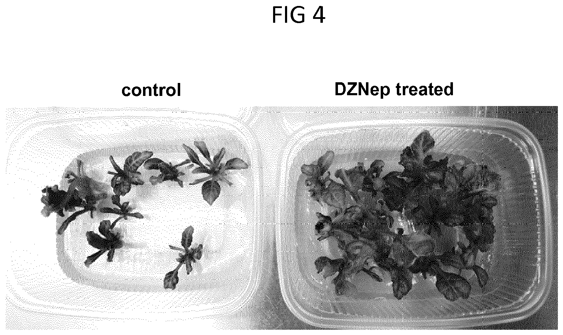

Beside the increased capability for shoot formation the inventors observed that shoots harvested from the control plates and from the plates supplied with DZNep and subsequently transferred into the container with normal growth medium (without DZNep), developed to plant with different biomass and vitality. Plants grown from DZNep-induced shoots look significantly bigger and more vital (see Figure 4). Thus, the DZNep treatment affects also positively the plant growth after regeneration.

## Claims

1. A method for inducing shoot formation from callus tissue of a plant, comprising the step of cultivating callus tissue in the presence of an inhibitor of the histone methyltransferase EZH2.

2. The method of claim 1, wherein the inhibitor of the histone methyltransferase EZH2 is 3-deazaneplanocin A (DZNep) or a salt or derivative thereof.

3. The method of claim 1 or 2, wherein the step of cultivating the callus tissue comprises
growing the callus tissue in a medium comprising the inhibitor of the histone methyltransferase EZH2, in particular DZNep, preferably in a concentration of 0.05 to 5.0 µM, and/or
introducing the inhibitor of the histone methyltransferase EZH2, in particular DZNep, into at least one cell of the callus tissue, for example via bombardment, electroporation or microinjection.

4. The method of any one of claims 1-3, wherein the callus tissue is provided by inducing callus formation from at least one plant cell under conditions promoting the growing of callus tissue.

5. A method for producing a transgenic plant, comprising the following steps:
(a) Inducing callus formation from at least one plant cell under conditions promoting the growing of callus tissue,
(b) Introducing into a plant cell to be used in step (a) and/or into a cell of the callus obtained in step (a) at least one nucleotide sequence of interest or at least one polypeptide of interest,
(c) Cultivating the callus tissue obtained from steps (a) and (b) according to the method of any one of claims 1-3, and
(d) Regenerating a transgenic plant.

6. A method of producing a genetically modified plant, comprising the following steps
(a) Inducing callus formation from at least one plant cell under conditions promoting the growing of callus tissue, and
(b) Modifying the genome of a plant cell to be used in step (a) and/or of a cell of the callus tissue obtained in step (a) by introducing into said cell a double stranded DNA break (DSB) inducing enzyme which preferably recognizes a predetermined site in the genome of said cell, and optionally a repair nucleic acid molecule, and/or a base editor fused to a catalytically impaired double stranded DNA break (DSB) inducing enzyme which preferably recognizes a predetermined site in the genome of said cell,
wherein the modification of said genome is selected from
i. a replacement of at least one nucleotide;
ii. a deletion of at least one nucleotide;
iii. an insertion of at least one nucleotide; or
iv. any combination of i.- iii.,
(c) Cultivating the callus tissue obtained after steps (a) and (b) according to the method of any one of claims 1-3, and
(d) Regenerating a genetically modified plant.

7. A method of producing a haploid or double haploid plant, comprising the steps
(a) Inducing callus formation from an immature male gametophyte or a microspore under conditions promoting the growing of callus tissue,
(b) Cultivating the callus tissue obtained in step (a) according to the method of any one of claims 1-3,
(c) Optionally conducting chromosome doubling, and
(d) Regenerating a haploid or double haploid plant.

8. A transgenic plant obtained or obtainable by the method of claim 5 or a progeny plant thereof.

9. A genetically modified plant obtained or obtainable by the method of claim 6, or a progeny plant thereof.

10. A haploid or double haploid plant obtained or obtainable by the method of claim 7 or a progeny plant thereof.

11. A plant cell or a seed of the plant of claim 8, wherein the plant cell or the seed comprises the at least one nucleotide sequence of interest as transgene.

12. A plant cell or a seed of the plant of claim 9, wherein the plant cell or the seed comprises the modification in the genome.

13. A plant cell or a seed of the plant of claim 10, wherein the plant cell or the seed comprises a haploid or double haploid set of chromosomes.

14. Use of an inhibitor of the histone methyltransferase EZH2, preferably DZNep, in a method for inducing shoot formation from callus tissue of a plant or in a method for indirect regeneration of a plant.

15. Use of an inhibitor of the histone methyltransferase EZH2, preferably DZNep, in a method for production of a transgenic plant cell, plant or seed, in a method for production of a genetically modified plant cell, plant or seed or in a method for production of a haploid or double haploid plant cell, plant or seed.
